# EUROPEAN PATENT APPLICATION

(11) **EP 1 852 501 A1**
(43) Date of publication of application: **07.11.2007**
(21) Application number: 07107354.8
(22) Date of filing: 02.05.2007
(51) Int. Cl.: C12N 5/06, A61K 35/12

(54) **Methods and compositions for stem cell therapies**

(30) Priority: 03.05.2006 US 797764 P
(71) Applicant: Moviglia, Gustavo Antonio, Buenos Aires 1245 (AR)
(72) Inventor: Moviglia, Gustavo Antonio, Buenos Aires 1245 (AR)
(74) Representative: Holmberg, Martin Tor

(57) **Abstract**

The present invention includes methods and compositions for the differentiation of stem cells and enhancement of stem cell therapies. In certain embodiments, the present invention includes methods and compositions of autoimmune T-cells reactive to the desired cell type or types cultured with stem cells to induce differentiation of the stem cells to the desired cell type or types. In certain other embodiments, the present invention includes methods of enhancing stem cell therapies by administration to a subject of autoimmune T-cells reactive to the cell type or types useful for cell therapies.

## Description

### FIELD OF THE INVENTION

The present invention relates to the fields of stem cell therapeutics, especially as related to autoimmune T cell induced differentiation of stem cells.

### BACKGROUND OF THE INVENTION

Stem cell bioengineering is a nascent technology that holds great promise for therapeutic treatment of a wide range of disorders; however, a fundamental problem remains to be solved which is how to control stem cell differentiation and lineage commitment *in vitro* so that the bioengineered stem cells may be used *in vivo.* Currently, detailed methods must be developed in part by trial and error using different media conditions supplemented with growth factors known to be involved in differentiation. A generalized method that could easily be adapted to generate a wide range of stem cell types would allow a multitude of therapeutic applications to be developed. Potential applications include: generating different types of neurons for the treatment of Alzheimer's disease, spinal cord injuries, amyotrophic lateral sclerosis, or Parkinson's disease; producing heart muscle cells for heart attack survivors; generating insulin-secreting pancreatic islet cells for the treatment of type-1 diabetes; and even generating hair follicle stem cells for the treatment of certain types of baldness. Stem cells that could be readily differentiated into desired cell types could also be useful for a number of tissue engineering applications such as the production of complete organs including livers, kidneys, eyes, hearts, or even parts of the brain. In addition, the ability to control stem cell proliferation and differentiation has applicability in *in vitro* drug testing, cancer research, and fundamental research on embryonic development.

Thus there is a need for a generalized method that may be used to induce differentiation of stem cells to a wide range of different cell types.

### SUMMARY

The present invention addresses these long felt needs by providing methods and compositions for enhancing stem cell therapies including compositions and methods of inducing stem cells to differentiate into a desired cell type by co-culturing stem cells with autoimmune T-cells reactive against the desired cell type and methods and compositions for administering autoimmune T-cells reactive against a desired cell type to a patient with damage to the desired cell type followed by administration of stem cells, preferably stem cells that have been induced to differentiate into the desired cell type by co-culture *in vitro* with the autoimmune T-cells reactive against the desired cell type. In a preferred embodiment of the present invention, the desired cell type may be CNS cells, preferably for treatment of chronic spinal cord injuries.

One aspect of the present invention includes methods of inducing a population of stem cell to differentiate into a target cell type where a population of stem cells capable of differentiating into the target cell type are contacted with a population of autoimmune T-cells reactive against the target cell type for a time period sufficient to induce differentiation of the population of stem cells into the target cell type. In certain preferred embodiments, the target cell type may be central nervous system cells, pancreatic cells, and heart muscle cells. In some embodiments, the population of stem cells may be mesenchymal stem cells, adipose-derived stem cells or hematopoietic stem cells.

Another aspect of the present invention includes methods of treating a subject with organ, tissue or cell damage wherein a population of stem cells capable of differentiating into the organ cells, tissue cells or cells that are damaged are contacted with a population of autoimmune T-cells reactive against the organ cells, tissue cells or cells for a time period sufficient to induce differentiation of the population of stem cell into a differentiated population of the organ cells, tissue cells or cells. A therapeutically effective amount of the differentiated population of the organ cells, tissue cells or cells are then administered to the subject. In certain preferred embodiments, a therapeutically effective amount of the population of autoimmune T-cells may be administered to the subject prior to administering the differentiated stem cells to the subject. In preferred embodiments, the damage is neural damage, more preferably spinal cord damage, amyotrophic lateral sclerosis, Parkinson's disease, or Alzheimer's disease. In preferred embodiments, the populations of stem cells are mesenchymal stem cells, adipose-derived stem cells or hematopoietic stem cells.

Another aspect of the present invention includes methods of treating a subject with organ, tissue or cell damage where a therapeutically effective amount of a population of autoimmune T-cells reactive to the organ cells, tissue cells or cells are administered to the subject followed by a therapeutically effective amount of a population of stem cells capable of differentiating into the cell type. In preferred embodiments, the population of stem cells has been induced to differentiate by contacting the stem cells with a population of autoimmune T-cells reactive against the organ cells, tissue cells or cells for a time period sufficient to induce differentiation of the population of stem cell into a population of the organ cells, tissue cells or cells before administering to the subject. In preferred embodiments, the damage is neural damage, more preferably spinal cord damage, amyotrophic lateral sclerosis, Parkinson's disease, or Alzheimer's disease. In preferred embodiments, the populations of stem cells are mesenchymal stem cells, adipose-derived stem cells or hematopoietic stem cells.

Yet another aspect of the present invention includes compositions which include a population of stem cells capable of differentiating into the target cell type in contact or co-cultured with a population of autoimmune T-cells reactive to at least one protein expressed by the target cell type. In preferred embodiments, the population of autoimmune T-cells is reactive to proteins expressed in central nervous system cells, pancreatic cells, or heart muscle cells. In certain preferred embodiments, the populations of stem cells are mesenchymal stem cells, adipose-derived stem cells or hematopoietic stem cells.

Still another aspect of the invention includes methods of isolating autoimmune T-cells reactive to an organ, tissue or cell type where a population of cells comprising mononuclear cells are isolated from a subject and the desired autoimmune T-cells are expanded within the population by contacting the population of cells with antigens obtained from the organ, tissue or cell type obtained from the same or related species. In preferred embodiments, the subject is human and the related species are human (autologous or allogeneic antigens), bovine, ovine, canine, or feline. In preferred embodiments, the autoimmune T-cells are then separated from the population by positive or negative selection.

### SUMMARY OF THE FIGURES

Figure 1: (A) shows the SEP of Patient 1 taken two months after the spinal cord injury, and (B) shows the SEP of Patient 1 taken six weeks after the first round of therapy.

Figure 2: (A) shows the MRI of Patient 1 taken three days after the spinal cord injury, and (B) shows the MRI of Patient 1 taken six weeks after the first round of therapy.

### DETAILED DESCRIPTION OF THE PREFERRED EMBODIMENTS

The present invention relates to novel methods and compositions that enhance stem cell therapies and includes several aspects. One preferred aspect are the compositions and methods of inducing stem cells to differentiate into a desired cell type by co-culturing stem cells with autoimmune T-cells reactive against the desired cell type. Another preferred aspect of the present invention is to administer autoimmune T-cells reactive against a desired cell type to a patient with damage to the desired cell type followed by administration of stem cells. In a preferred variation, the stem cells administered will have been induced to differentiate into the desired cell type by co-culture in vitro with the autoimmune T-cells reactive against the desired cell type. In a preferred embodiment of the present invention, the desired cell type may be CNS cells, preferably for treatment of chronic spinal cord injuries.

Recent observations have suggest that, although to a lesser extent, superior vertebrates have regenerative processes similar to those, more significant and effective, regenerative processes found in inferior vertebrates after traumatic or ischemic lesions of the central nervous system.

The studies performed on autopsies of female patients who had received transplants of bone marrow from histocompatible male donors showed that 1 to 2% of the CNS cells in the lesion site had a karyotype with a Y chromosome. This observation indicated that the transplanted bone marrow MSCs had differentiated into CNS cells at the lesion site. This and other similar studies have showed that stem cell therapies hold some promise for treatment of spinal cord injuries.

Simultaneously, other clinical data has shown that traumatic lesions in heart and CNS are followed by a significant mobilization in blood of CD133 cells. CD133 is a marker of stem cells in bone marrow and fat tissue. This mobilization reaches a peak within a week of the time the injury occurs.

In 1999, Schwartz established that the immune system plays a fundamental role in the repair process. She observed that after experimental spinal cord or optic nerve injury, an inflammatory reaction occurs. Macrophages and lymphocytes are predominant in this reaction. The lymphocytes are autoreactive and similar to those which produce encephalitis or autoimmune demyelization. But, this reaction is limited in time.

The intensity of this phenomenon is directly proportional to the regenerative capacity presented by each species. Amphibians, such as toads and salamanders, present an inflammatory infiltration ten times higher than that presented by rats after the same degree of spinal cord or optic nerve injury.

Schwartz's group, helped by Irun Cohen et al, has experimentally proved that the exogenous administration of anti-myelin lymphocytes to mammals enabled the restoration of those lesions. This phenomenon could only take place in a window period of fifteen days after the injury. Studies by Hohlfeld and collaborators have shown that these anti-myelin autoimmune T-cells secrete neurotrophic factors of the neurotrophin family, such as Brain Growth Factor and Nerve Growth Factor. All of these phenomena support the idea that the lymphocytes in the lesion site act as inducers of regeneration through the transdifferentiation of MSCs which were attracted to the site due to the inflammation process.

In one aspect, the present invention included a new therapeutic approach to promote the regeneration of CNS tissue at the site of chronic spinal cord injuries. The new therapeutic approach is based on the combination of a treatment with autologous autoimmune T-cells reactive against spinal cord cells, followed by the administration (systemic or through selective catheterization) of autologous MSCs transdifferentiated to CNS cells. Example 5 below describes the results of administration of this new therapeutic approach to two patients.

While not wanting to be limited by theory, the rationale for treatment with CD3⁺ CD25⁻ lymphocytes reactive against CNS cells is based upon the observation that such autoimmune T-cells have strong specificity to attach to CNS capillaries, cross the blood-brain barrier and infiltrate the damaged area increasing the local inflammation. This specific and local inflammation generates a neurotrophin and cytokine microenvironment which in turn attracts circulating MSCs and promote their transdifferentiation into CNS cells to repair the damaged area.

### Source of the Stem Cells

Stem cells for use with the present invention may be obtained from any source. By way of example, pluripotent stem cells can be isolated from the primordial germinal ridge of the developing embryo, from teratocarcinomas, and from non-embryonic tissues, including but not limited to the bone marrow, brain, liver, pancreas, peripheral blood, placenta, skeletal muscle, and umbilical cord blood. Stem cells display a number of common properties. They typically display high levels of alkaline phosphatase enzyme activity (Shamblott et al. 1998, Proc Natl Acad Sci USA 95:13726-13731). They also usually express high levels of the telomerase enzyme, a ribonucleoprotein that catalyzes the addition oftelomere repeats to chromosome ends. This activity maintains the chromosome length and is correlated with cell immortality (Odorico et al 2001, Stem Cells 19:193-204).

The methods and compositions of the present invention may be used with and include embryonic stem cells. Embryonic stem cells are typically derived from the inner cell mass of blastocyst-stage embryos (Odorico et al. 2001, Stem Cells 19:193-204; Thomson et al. 1995. Proc Natl Acad Sci USA. 92:7844-7848.; Thomson et al. 1998. Science 282:1145-1147). The distinguishing characteristic of these cells are (i) their ability to be cultured in their non-differentiated state and (ii) their capacity to give rise to differentiated daughter cells representing all three germ layers of the embryo and the extra-embryonic cells that support development. Embryonic stem cells have been isolated from other sites in the embryo.

Embryonic stem cells of human origin typically express cell surface markers including but not limited to stage-specific embryonic antigens 3 and 4 (SSEA-3 and SSEA-4), high molecular weight glycoproteins TRA-1-60 and RA-1-81, and alkaline phosphatase (Amit M et al. 2000, Dev Biol 227:271-278; Odorico et al, Stem Cells 19:193-204). In their undifferentiated state, the embryonic stem cells retain their ability to express the transcription factor October 4; with differentiation commitment, the level of October 4 decreases (Reubinoff et al, 2000, Nature Biotechnology 18:399-404.; Schuldiner et al. 2000, Proc Natl Acad Sci USA 97:11307-11312).

In addition to the preferred method of inducing differentiation with autoimmune T-cells as described herein, embryonic stem cells may be induced to undergo lineage specific differentiation in response to a panel of cytokines. Representative examples from the literature are cited below but the list is not intended to be comprehensive or exhaustive. Transforming growth factor β1 and activin A inhibit endodermal and ectodermal differentiation while promoting mesodermal lineages such as skeletal and cardiac muscle (Schuldiner et al. 2000, Proc Natl Acad Sci USA 97:11307-11312). Retinoic acid, basic fibroblast growth factor, bone morphogenetic protein 4, and epidermal growth factor induce both ectodermal (skin, brain) and mesodermal (chondrocyte, hematopoietic) lineages (Schuldiner et al. 2000). Other factors, such as nerve growth factor and hepatic growth factor, promote differentiation along all three embryonic lineages (ectodermal, endodermal, and mesodermal). Still other factors such as platelet derived growth factor promote glial cell differentiation (Brustle et al 1999, Science 285(5428):754-6). Transplantation of the embryonic stem cells into skeletal muscle tissue or other tissue site of immunodeficient mice leads to the development of a teratocarcinoma, exhibiting the differentiation of gut-like structures, neural epithelium, cartilage, striated muscle, glomeruli and other tissue types (Thomson et al 1998, Curr Top Dev Biol 38:133-165; Amit et al. 2000, Dev Biol 227:271-278; Reubinoff et al. 2000, Nature Biotechnology 18:399-404). Alternatively, the embryonic stem cells differentiate into cells derived from all three germinal layers when cultured in vitro as embryoid bodies (Itskovitz-Eldor J et al. 2000, Mol Med 6:88-95; Reubinoffet al. 2000, Nature Biotechnology 18:399-404).

Some of the current methods for the isolation and maintenance of embryonic and other stem cell lines rely on the use of murine embryonic fibroblasts (MEF). Prior to co-culture, the MEF are irradiated (levels of between 35-50 gray) to reduce cell proliferation without compromising metabolic function (Shamblott et al. 1998, Proc Natl Acad Sci USA 95:13726-13731; Amit et al 2000, Dev Biol 227:271-278). Embryonic stem cells are isolated from the inner cell mass from blastocyst stage embryos by immunosurgery (Reubinoff et al. 2000, Nature Biotechnology 18:399-404; Thomson et al. 1998, Science 282:1145-1147; Odoricoet al 2001, Stem Cells 19:193 -204). The zona pellucida is digested with pronase, the inner cell mass is isolated by immunosurgery with an antihuman serum antibody followed by exposure to guinea pig complement, and the resulting cells plated onto the irradiated MEF feeder layer culture (Reubinoff et al. 2000, Nature Biotechnology 18:399-404; Thomson et al. 1998, Science 282:1145-1147). Alternatively, cells are isolated from the gonadal ridges and mesenteries of 5 to 9 week old post-fertilization human embryos following mechanical disaggregation and trypsin/EDTA digestion or hyaluronidase/collagenase IV/Dnase digestion and subsequent plating onto an MEF feeder layer (Shamblott et al. 1998, Proc Natl Acad Sci USA 95:13726-13731). Cultures are maintained in the presence of Dulbecco's Modified Eagle's Medium (no pyruvate, high glucose formulation), Knock Out Dulbecco's Modified Eagle's Medium or equivalent medium supplemented with 15-20% fetal bovine serum or 15-20% Knock Out SR (Gibco/BRL, Gaithersburg Md.), a serum replacement optimized for embryonic stem cell growth (Price et al WO98/30679), 0.1 mM nonessential amino acids, 0.1 mM 2-mercaptoethanol, 2 mM glutamine, antibiotics, and the addition of up to 2,000 units/ml of human recombinant leukemia inhibitory factor (LIF), up to 4 ng/ml of human recombinant basic fibroblast growth factor, and up to 10 µM forskolin (Amit et al. 2000, Dev Biol 227:271-278; Reubinoff et al. 2000, Nature Biotechnology 18:399-404; Shamblott et al. 1998, Proc Natl Acad Sci USA 95:13726-13731; Thomson et al. 1998, Science 282:1145-1147).

The frequency of embryonic stem cell clones has been noted to increase several-fold with the use of serum replacements (Amit et al. 2000, Dev Biol 227:271-278). The presence of basic fibroblast growth factor is required for the continued undifferentiated proliferation of the clonal embryonic stem cells. The combined presence of bFGF, LIF, and forskolin is associated with the development of tight, compacted multicellular human embryonic stem cell colonies as opposed to flattened, loose colonies seen with other primates (rhesus) (Shamblott et al. 1998, Proc Natl Acad Sci USA 95:13726-13731). After 9 to 15 days, individual colonies are dissociated into clumps by exposure to calcium and magnesium free phosphate buffered saline with 1 mM EDTA or other divalent cation chelator, by exposure to dispase (10 mg/ml), or by mechanical dissociation with a micropipette (Thomson J A, Itskovitz-Eldor J, Shapiro S S, et al. 1998. Embryonic stem cell lines derived from human blastocysts. Science 282:1145-1147). The resulting cells or cell clumps are sequentially plated onto irradiated mouse embryonic fibroblasts in fresh medium (Thomson J A et al. 1998, Science 282:1145-1147; Reubinoff et al. 2000, Nature Biotechnology 18:399-404). Clones are expanded on the order of every 7 days and display a doubling time of approximately 36 hours (Amit et al. 2000, Dev Biol 227:271-278). Subsequent passage of the clones is carried out by repeating the cell disruption procedure (digestion, micropipette manipulation) and by plating the resulting cells on irradiated MEFs (Amit et al. 2000, Dev Biol 227:271-278).

Methods of culturing stem cells without MEFs may be found in U.S. Patent Publ. 20030152558 and 20020090723 (both of which are hereby incorporated by reference for all that they teach with particular reference to the disclosed methods of culturing stem cells).

In preferred embodiments of the present invention, adult stem cells are used, more preferably mesenchymal stem cells (MSCs). MSCs are pluripotent cells found in bone marrow and periosteum, and they are capable of differentiating into various mesenchymal or connective tissues. For example, such bone-marrow derived stem cells can be induced to develop into myocytes upon exposure to agents such as 5-azacytidine (Wakitani et al., Muscle Nerve, 18 (12), 1417-26 (1995)). It has been suggested that such cells are useful for repair of tissues such as cartilage, fat, and bone (see, e.g., U.S. Pat. Nos. 5,908,784, 5,906,934, 5,827,740, and 5,827,735, which are hereby incorporated by reference in their entirety), and that they also have applications through genetic modification (see, e.g., U.S. Pat. No. 5,591,625, which is hereby incorporated by reference in its entirety).

Another example of an adult stem cell is adipose-derived adult stem cells (ADSCs) which have been isolated from fat, typically by liposuction followed by release of the ADSCs using collagenase. ADSCs are similar in many ways to MSCs derived from bone marrow, except that it is possible to isolate many more cells from fat. These cells have been shown to differentiate into bone, fat, muscle, cartilage, and neurons. These cells have been recently used to successfully repair a large cranial defect in a human patient. An improved method of isolation has been described in U.S. Patent Publ. 20050153442 (which is hereby incorporated in its entirety with particular reference to the methods of isolating ADSCs and inducing ADSCs to differentiate into desired cell types).

U.S. Pat. No. 5,851,832 reports multipotent neural stem cells obtained from brain tissue. U.S. Pat. No. 5,766,948 reports producing neuroblasts from newborn cerebral hemispheres. U.S. Pat. Nos. 5,654,183 and 5,849,553 report the use of mammalian neural crest stem cells. U.S. Pat. No. 6,040,180 reports in vitro generation of differentiated neurons from cultures of mammalian multipotential CNS stem cells. WO 98/50526 and WO 99/01159 report generation and isolation of neuroepithelial stem cells, oligodendrocyte-astrocyte precursors, and lineage-restricted neuronal precursors. U.S. Pat. No. 5,968,829 reports neural stem cells obtained from embryonic forebrain and cultured with a medium comprising glucose, transferrin, insulin, selenium, progesterone, and several other growth factors.

Primary liver cell cultures can be obtained from human biopsy or surgically excised tissue by perfusion with an appropriate combination of collagenase and hyaluronidase. Alternatively, EP 0 953 633 A1 reports isolating liver cells by preparing minced human liver tissue, resuspending concentrated tissue cells in a growth medium and expanding the cells in culture. The growth medium comprises glucose, insulin, transferrin, T₃, FCS, and various tissue extracts that allow the hepatocytes to grow without malignant transformation. The cells in the liver are thought to contain specialized cells including liver parenchymal cells, Kupffer cells, sinusoidal endothelium, and bile duct epithelium, and also precursor cells (referred to as "hepatoblasts" or "oval cells") that have the capacity to differentiate into both mature hepatocytes or biliary epithelial cells (L. E. Rogler, Am. J. Pathol. 150:591, 1997; M. Alison, Current Opin. Cell Biol. 10:710,1998; Lazaro et al., Cancer Res. 58:514, 1998).

U.S. Pat. No. 5,192,553 reports methods for isolating human neonatal or fetal hematopoietic stem or progenitor cells. U.S. Pat. No. 5,716,827 reports human hematopoietic cells that are Thy-1 positive progenitors, and appropriate growth media to regenerate them in vitro. U.S. Pat. No. 5,635,387 reports a method and device for culturing human hematopoietic cells and their precursors. U.S. Pat. No. 6,015,554 describes a method of reconstituting human lymphoid and dendritic cells.

The present invention can be practiced using stem cells of any vertebrate species. Included are stem cells from humans; as well as non-human primates, domestic animals, livestock, and other non-human mammals.

### Preparation of the Autoimmune T-Cells

The autoimmune T-cells of the present invention may be isolated by standard techniques for the isolation of lymphocytes. Example 1 describes a typical protocol that includes isolation and expansion of autoimmune T-cells reactive against a desired organ, tissue or cell type. Autoimmune T-cells may be isolated from any subject presenting autoimmune T-cells reactive against the desired organ, tissue, or cell type including healthy subjects. Preferably the subject will have damage to the desired organ, tissue or cell type, thus increasing the circulating autoimmune T-cells. Examples include autoimmune related disorders such Type 1 diabetes and non-autoimmune autoimmune diseases such as amyotrophic lateral sclerosis (ALS) or spinal cord injury The autoimmune T-cells may be allogeneic to the subject to be treated, but are preferably autologous. After isolation of a population of autoimmune T-cells, the population of cells is preferably expanded against antigens from the desired cell type. The antigen may be one or more purified proteins known to be involved in protective immunity such as myelin basic protein or Cop-1 for neural disorders or other organ, tissue or cell specific antigen may be used such as disclosed in U.S. Patent Publ. 20050220803 (which is hereby incorporated by reference for all that it teaches with particular reference to peptides for treatment of non-autoimmune disorders that may be used to expand autoimmune T-cells herein). In addition, the antigen may be crude or partially purified lysates of the desired organ, tissue or cell type. Such lysates may be isolated from the same species as the subject to be treated or preferably from different species to decrease the risk of disease transfer.

### Uses of the Autoimmune T-Cells for Transdifferentiation

The isolated autoimmune T-cells of the present invention may be used for *in vitro* co-culture with any of the stem cells to induce differentiation of the stem cells into the desired organ, tissue or specific cell type. Any standard cell culture media may be used that is compatible with both the stem cell used and the autoimmune T-cells. The cells should be co-cultured for a sufficient time to induce differentiation of the stem cells, preferably at least 12 hours, at least 24 hours, at least 48 hours or even at least 72 hours. One of skill in the art can readily determine the time for co-culture by monitoring the stem cells by morphology, by histochemical assay, by immunohistochemical assay or any other assay sufficient to distinguish between the undifferentiated stem cell and the differentiated stem cell. The co-culture may be subject to additional separation or purification to remove the autoimmune T-cells and/or the undifferentiated stem cells prior to use of the differentiated stem cells. The differentiated stem cells may be used for any application involving stem cells including, without limitation, the uses described under "Uses of the Differentiated Stem Cells for Stem Cell Therapy" below.

### Uses of the Autoimmune T-cells for Stem Cell Therapy

In addition to their use in differentiating stem cells, autoimmune T-cells may be used in conjunction with stem cell therapies to enhance the stem cell therapy. The autoimmune T-cells may be used in conjunction with any stem cells, preferably stem cells that have been induced to differentiate with autoimmune T-cells. While not wanting to be limited to theory, as discussed above, the autoimmune T-cells act to prepare a microenvironment that conducive to stem cell differentiation and repair of damaged organ, tissue and cells. Given T-cells ability to infiltrate sites of inflammation, the autoimmune T-cells may be administered by any available technique for delivery of cells to an area of damage including, by way of example, systemic administration, catheterized endovascular administration, direct injection, and implantation of measured release device.

Autoimmune T-cells increase their inflammatory activity during the three days following administration to a subject. Thus, a preferred time for administration of the stem cells is at least about 24 hours after, at least about 32 hours after, at least about 48 hours after, or at least about 72 hours after the autoimmune T-cell administration. An even more preferred time for administration of the stem cells is at approximately 48 hours after administration of the autoimmune T-cells, when the autoimmune T-cells still need another 24 hours to reach their peak activity.

### Uses of the Differentiated Stem Cells for Stem Cell Therapy

The differentiated stem cells of the present invention may be used as any other differentiated stem cell. By way of example, differentiated stem cells of the present invention can be used for tissue reconstitution or regeneration in a human patient in need thereof The differentiated stem cells are administered in a manner that permits them to graft to the intended tissue site and reconstitute or regenerate the functionally deficient area. In preferred embodiments, the differentiated stem cells are administered in conjunction with the autoimmune T-cells to enhance the therapeutic effect. A preferred method of administration is delivery through the peripheral blood vessel of the subj ect, given that the stem cells are preferentially attracted to the damaged areas. Such delivery overcomes the failures observed by others using systemic administration of stem cells because the autoimmune T-cells increase the inflammation which in turn increases the stem cells attraction to the damaged area. An even more preferred form of administration is by selective catheterization at or around the site of damage, which can lead to almost complete delivery of the stem cells into the damaged area.

In a preferred embodiment, CNS differentiated stem cells are delivered to a patient by vascular delivery as discussed in Example 5 below. In other embodiments, CNS differentiated stem cells are transplanted directly into parenchymal or intrathecal sites of the central nervous system, according to the disease being treated. Grafts are done using single cell suspension or small aggregates at a density of 25,000-500,000 cells per µL (U.S. Pat. No. 5,968,829). The efficacy of neural cell transplants can be assessed in a rat model for acutely injured spinal cord as described by McDonald et al. (Nat. Med. 5:1410, 1999). A successful transplant will show transplant-derived cells present in the lesion 2-5 weeks later, differentiated into astrocytes, oligodendrocytes, and/or neurons, and migrating along the cord from the lesioned end, and an improvement in gate, coordination, and weight-bearing.

Neural differentiated stem cells of the present invention may also be used for treatment of acute or chronic damage to the nervous system. For example, excitotoxicity has been implicated in a variety of conditions including epilepsy, stroke, ischemia, Huntington's disease, Parkinson's disease and Alzheimer's disease. Certain differentiated stems cells of the present invention may also be appropriate for treating dysmyelinating disorders, such as Pelizaeus-Merzbacher disease, multiple sclerosis, leukodystrophies, neuritis and neuropathies. Appropriate for these purposes are differentiated stem cell cultures enriched in oligodendrocytes or oligodendrocyte precursors to promote remyelination.

Hepatocytes and hepatocyte stem cells differentiated using the methods of the present invention can be assessed in animal models for ability to repair liver damage. One such example is damage caused by intraperitoneal injection ofD-galactosamine (Dabeva et al., Am. J. Pathol. 143:1606, 1993). Efficacy of treatment can be determined by immunohistochemical staining for liver cell markers, microscopic determination of whether canalicular structures form in growing tissue, and the ability of the treatment to restore synthesis of liver-specific proteins. Liver cells can be used in therapy by direct administration, or as part of a bioassist device that provides temporary liver function while the subject's liver tissue regenerates itself following fulminant hepatic failure.

The efficacy of cardiomyocytes differentiated using the methods of the present invention can be assessed in animal models for cardiac cryoinjury, which causes 55% of the left ventricular wall tissue to become scar tissue without treatment (Li et al., Ann. Thorac. Surg. 62:654, 1996; Sakai et al., Ann. Thorac. Surg. 8:2074, 1999, Sakai et al., J. Thorac. Cardiovasc. Surg. 118:715, 1999). Successful treatment will reduce the area of the scar, limit scar expansion, and improve heart function as determined by systolic, diastolic, and developed pressure. Cardiac injury can also be modeled using an embolization coil in the distal portion of the left anterior descending artery (Watanabe et al., Cell Transplant. 7:239, 1998), and efficacy of treatment can be evaluated by histology and cardiac function. Cardiomyocyte preparations embodied in this invention can be used in therapy to regenerate cardiac muscle and treat insufficient cardiac function (U.S. Pat. No. 5,919,449 and WO 99/03973).

### PREFERRED METHOD

### Example 1.

Effector T-Cells were isolated using the following protocol. Patients suffering from spinal cord lesions and Amyotrohpic Lateral Sclerosis were subject to apheresis using a Cobe Spectra, Gambro, apheresis machine. Two blood volumes from each patient was subject to the buffy coat program to obtain a buffy coat suspension with the following composition: mono-nuclear cells (MNCs): 85%, red blood cells (RBCs): 1.8 x 10⁶/µl, and platelets: 6 x 10⁵/µl. To purify the MNCs, the buffy coat suspension was seeded on a Ficoll-Hypaque gradient (1.077 density). After centrifugation, the MNCs recovered were washed in DBSS without Ca²⁺ or MG²⁺. The composition of the MNCs after the Ficoll-Hypaque gradient was: MNCs: 98%, RBCs: 0.2 x 10⁶/µl, and platelets: 1 x 10⁴/µl.

The MNC suspension was cultured for four days in DMEM enriched with a partial hydrolysate of bovine brain and a partial hydrolysate of bovine spinal cord (Laboratorios Villar, Argentina). After two days in cell culture, interleukin-2 (IL2) (Laboratorio Gautier, Argentina) was added to a final concentration of 100 IU/ml.

At day five, CD3⁺ lymphocytes were isolated by negative selection using a Monoclonal Antibody (MAB) cocktail against the undesired cells (MABs against CD14, CD16, CD 19, CD 56 and Glycophorin A). The first incubation with the MAB cocktail was followed by a second incubation with a solution of anti-MAB antibodies attached to a paramagnetic Teflon bead (Stem Sep kit, Stem Cell Technology. Vancouver, Canada).

After the second incubation, the lymphocytes were passed through a powerful magnetic field, which allowed the CD3⁺ cells to pass through while retaining the rest of the cells. The CD3⁺ cells were enriched to a purity of approximately 96%.

This CD3⁺ enriched suspension was then incubated with an anti-CD25 MAB solution (Stem Sep kit, Stem Cell Vancouver, Canada). The CD3⁺ CD25⁻ lymphocytes were separated from the CD3⁺ CD25⁻ lymphocytes using paramagnetic Teflon beads couples to anti-MAB antibodies. The proportion of CD3⁺ CD25⁻ lymphocytes versus CD3⁺ CD25⁻ lymphocytes obtained from this protocol depended on the particular condition of each donor.

### Example 2.

Bone marrow samples were obtained through needle aspiration of approximately 600 cc. of iliac crest bone marrow. Such samples consisted of approximately 8-10 x 10⁹ total MNCs. The composition of such samples was: 20% lymphocytes, 5% MID cells; 75% granulocytes; platelets 1.6 x 10⁵/µl and RBCs 4-5 x 10⁶/µl. Assaying such samples with anti-CD133 antibodies (CD133 is a marker for stem cells) revealed approximately 1.5 - 2 x 10⁸ total CD133 expressing cells.

The MSCs were purified from the bone marrow cell suspension through negative selection using a MAB cocktail (Rosette Sep Kit, Stem Cell Technology. Vancouver, Canada) and a Ficoll- Hypaque gradient. The unprocessed bone marrow suspension was concentrated by centrifugation at 300 g. The cells were suspended in a MAB cocktail composed of MAB against CD3, CD4, CD19, CD38, CD66b and Glycophorin A. The MAB cocktails cross-linked the undesired cells and the red blood cells (RBC) in the suspension forming immune rosettes. The cross-linking increased the density of the undesired cells so that they pelleted along with the free RBCs when centrifuged over a buoyant density medium such as Ficoll Hypaque (1.077 density). After this incubation, MSCs were not cross-linked and therefore were easily collected as a highly enriched population at the interphase between the plasma and the buoyant density medium. The cross-linking and Ficoll Hypaque gradient resulted in an 8-fold the concentration of MSC (the total yield was 1.5 to 2 x 10⁸ cells).

The purified MSCs were then cultured in Mesencult medium (Stem Cell Technology, Vancouver Canada) for 4-6 weeks at a concentration of 1 x 10⁶ cells per ml, in 25 mm² plastic Petri dishes at 37°C and at an atmosphere of O₂ 95%, CO₂ 5% in a Thermo Forma ® CO₂ incubator. The culture monitored using a phase control inverted microscope (Nikon Eclipse TS 100). MSCs reached semi-confluence after approximately 15 days. Once the MSCs reached semi-confluence, the cells were removed and re-seeded on fresh plastic Petri dishes. This process was repeated twice during the 4-6 week period.

### Example 3 - Differentiation of MSCs into CNS Cells.

MSCs were induced to differentiate into central nervous system cells by co-culturing with autoimmune T-cells reactive against central nervous system cells. MSCs were prepared according to the protocol in Example 2. After 4 to 6 weeks of culture when MSCs reached semi-confluency for the third time, the MSCs were removed and reseeded on 22 mm² cover slips in two 6-well plates. After culturing for fifteen days, the twelve wells were divided into three different groups of four wells each.

The first group was co-cultured with unselected mononuclear cells from the patient as a control. The second group was incubated with the CD3⁺ CD25⁻ lymphocytes from the patient prepared according to the protocol in Example 1. The third group was incubated with and CD3⁺ CD25⁺ lymphocytes from the patient prepared according to the protocol in Example 1.

### Example 4 - differentiation of MSCs into Other Cell Types.

Circulating autoimmune T-Cells reactive against central nervous system cells, heart cells, and pancreatic cells were isolated from patients with chronic damage caused by brain hypoxia were isolated using the method detailed in Example 1. Autologous MSCs were isolated using the method detailed in Example 2. The autoimmune T-Cells in each of the three categories were co-cultured with MSCs as follows:
Group 1: MSCs + autoimmune T-Cells reactive against pancreatic cells.
Group 2: MSCs + autoimmune T-Cells reactive against heart cells.
Group 3: MSCs + autoimmune T-Cells reactive against central nervous system cells.
Group 4: MSCs + lymphocytes not selected for reactivity (control).

The cells were co-cultured for two days and then examined for differentiation of the MSCs. The following cell types were observed in each of the four groups:
Group 1: A population of the MSCs had transformed into an array of cells in a cubical alignment resembling glandular adenoma. The cytoplasm of the cells in the array showed several secretory granules, but not exocytosis of the granules was observed.
Group 2: Two differentiated cell types were observed. The first class of cells presented a capillary endothelial shape. The second class of cells presented a typical myocardial structure, intercalated disks and myocardial fibrillar differentiation. Under a phase contrast microscope, a few of the cells were observed to contract spontaneously.
Group 3: A population of the MSCs had differentiated into a spindle shape. This population of cells.
Group 4: The MSCs in the control group retained their undifferentiated morphology.

Histochemical and immunhistochemical staining assays were performed and the assays confirmed the identification of the differentiated cell types based upon morphology.

### Example 5 - Treatment of spinal cord injuries.

Two patients were treated for spinal cord lesions using the autoimmune T-cells in combination with differentiated MSCs. A neurologist performed a physical evaluation of the patients and analyzed their MRI, angio MRI and somatosensory evoked potentials (SEPs) to determine the type of lesion, vascular access of the lesion site and to assess their functional deficit. Informed consent was obtained from the two participant patients after the nature and possible consequences of the studies were explained to them.

### Intravenous Infusion of CD3⁺ CD25⁻ AT against CNS proteins

As a first step in the treatment, the patient received an infusion of autoimmune T-cells prepared according to Example 1. A suspension of CD3⁺ CD25⁻ autoimmune T-cell reactive against CNS proteins was infused at a dose of 5 to 10 x 10⁸ cells/m² suspended in 500 cc ringer solution for eight (8) hours (at a rate of 63 ml/ hour) using an appropriate Peristaltic pump.

### Selective Endovascular Cell Implant of MNC or NSC

Forty eight hours after the infusion of autoimmune T-cells, the patient received CNS differentiated MNCs prepared according to Example 2. The femoral artery was punctured 3 cm below the inguinal crease using Judkins' technique. A Judkins catheter RCA 2 was placed through a 6 French introducer needle. The heparinization was performed locally in the puncture site.

The Adamkievicz artery or first intercostal artery was later catheterized as necessary depending upon the patient lesion site. The vertebral arteries were selectively catheterized. The catheter was set up in the dorsal area so as to reach the corresponding medullar area. The CNS differentiated stem cell suspension, in a total volume of 10 ml, was injected into each artery. The infusion pressure did not exceed 4.5 atmospheres.

Once this procedure was completed, the catheter and the arterial guide were removed and hemostasis was performed through local compression. No severe complications were observed. The patients remained in observation for 12 hours.

### Results - Patient 1

Patient 1 was a nineteen-year-old man who had suffered a car accident eight months before treatment. He presented paraplegia and his sensitive level corresponded to his sixth thoracic metamere (T6).

The patient had undergone two reparatory surgeries with poor results. After the second surgery the patient presented an infection in his prosthesis. Due to this infection he received antibiotics for a year.

The patient received intense neurorehabilitation during this period. After six months, since he was considered a chronic patient, he shifted to a less important neurorehabilitation program, thus developing severe muscle atrophy in his pelvic girdle and lower limbs. The patient presented little ability to fixate his trunk and thus was unable to rotate or make the movements necessary for his intimate hygiene. He was, however, able to carry out simple tasks such as feeding or brushing his teeth without assistance.

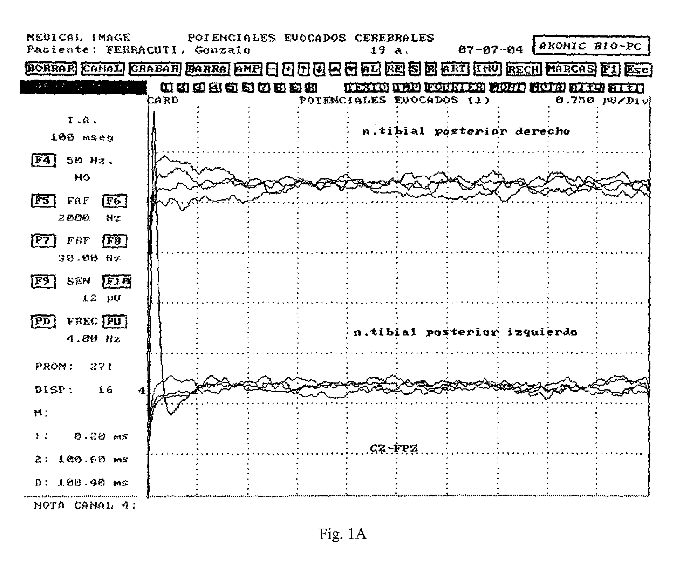

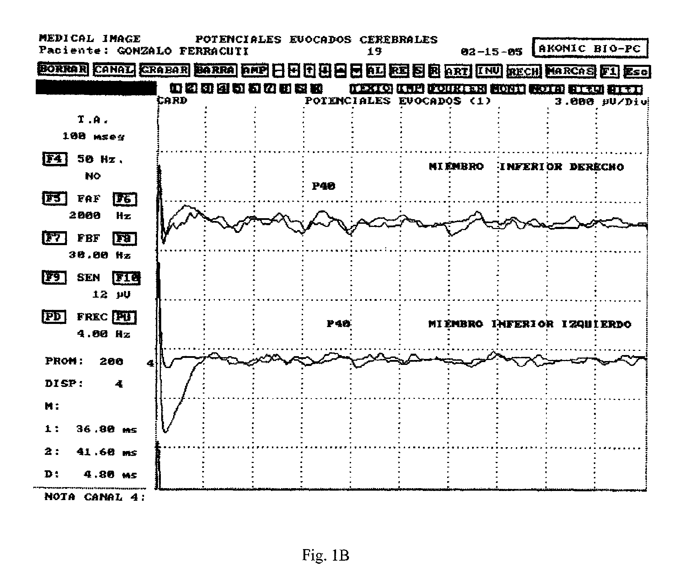

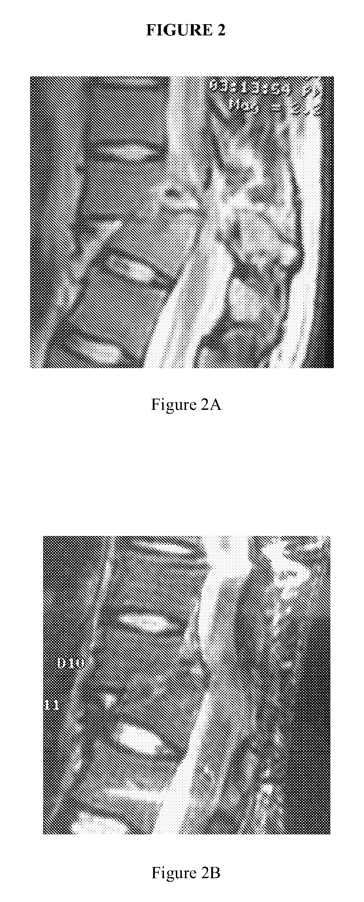

Two months after his first treatment with autoimmune T-cells followed by CNS differentiated MSCs, both his spine MRI and evoked potentials showed a clear improvement. A SEP (somatosensory evoked potentials) of the posterior tibial nerves at ankle level of both legs had been performed on the patient prior to treatment. This study showed no cortical arrival. A SEP of the same area performed after treatment showed a significant improvement in wave reproducibility and a normalization of the arrival rate to spinal cord and brain cortex (N 20 and P 40) (See Figures 1A and 1B) at 39.0 microseconds. The MRI showed a significant increase of the transversal diameter of the spinal cord (See Figures 2A and 2B). Although the patient presented a reappearance of spontaneous muscle contractions from the pelvic girdle to the knee, these contractions did not represent coordinated, effective movements. These new progress signs induced us to reinstitute a new intensive neurorehabilitation program. After three days, Patient 1 was able to move his lower limbs against gravity supported by the movements in his quadriceps for the first time since his accident.

During the following three months, Patient 1 presented progressive improvement that slowly reached a plateau. After the plateau, Patient 1 received a second round of therapy with autoimmune T-cells followed by CNS differentiated MSCs.

After the second round of therapy Patient 1's condition improved as he had better coordination in all his movements and developed the ability to walk helped by two canes and short prostheses. Currently, the patient presents a motor and sensitive levels corresponding to his first sacral metamere (S1).

At present Patient 1 is continuing with his neurorehabilitation program and will receive a third round of therapy with autoimmune T-cells followed by CNS differentiated MSCs. No adverse events have been detected to date.

### Results - Patient 2

Patient 2 is a twenty-one year old woman who had suffered a car accident thirty months before she started treatment.

Patient 2 suffered a spinal cord injury between her third and fifth cervical vertebrae (C3 - C5). Patient 2 presented no complications during her reparatory surgery. During the following two months the affected area extended to her seventh cervical vertebra (C7). Patient 2 presented a severe quadriplegia according to her lesion in her third cervical vertebra (C3). Patient 2's sensitivity level corresponded to her second cervical metamere (C2).

Patient 2 followed an intensive neurorehabilitation plan for twenty eight months. This program enabled Patient 2 to produce slight movements with her hands. These movements were not effective since Patient 2 was unable to hold an object. Patient 2 could, however, hold her head up, with some effort.

At the beginning of her therapy, Patient 2 presented total, severe muscle atrophy in upper limbs, back, trunk and lower limbs. Patient 2 required the use of two belts to remain seated in her chair. She was not able to brush her teeth, eat or perform those tasks necessary for her intimate hygiene by her own means.

After her first round of therapy with autoimmune T-cells followed by CNS differentiated MSCs, Patient 2 display signs of recovery that could be observed in her SEP, MRI and physical functions. The most significant change observed in her SEP was the latency changes in the N20, which used to arrive at 50 microseconds, as shown in the SEP performed on Patient 2 prior to treatment, and eight weeks after the first round of therapy this had improved to 28 microseconds. Due to this observation, it was believed that there had been remyelinization at the site of injury. The MRI showed an increase of the transversal diameter owed partially to certain inflammation in the lesion.

After the first round of therapy, Patient 2 had acquired the ability to hold her head, move her upper limbs, brush her own teeth and eat using special cutlery, paint and write helped by a wrist splint designed for assisting grip and pinch functions in the hand through the stabilization of the joint at about 30° dorsiflexion.

Although the patient currently presents voluntary movements in her trunk and lower limbs, her motor and sensitive levels correspond to her first and second thoracic metameres (T 1 and T 2). No adverse events were detected. A second round of therapy is planned.

### Conclusions

The important recovery of sensitivity and motor control observed in both patients, as well as the modifications observed in the SEPs, indicate that recovery of both glial cells and neuron fibers took place in the previously injured area. This recovery was coincident with the clinical progress observed in both patients. Patient 1 recovered effective and coordinated motor function after starting neurorehabilitation suggesting that the neurorehabilitation improved recovery the motor-neuron anagram of the CNS.

The rehabilitation program that Patient 1 received for 8 months prior to treatment was based on the development of those muscles which remained innervated after the accident. These muscles could compensate for the lost motor control as far as was possible (e.g. using the movements in his trunk, the patient could move his lower limbs through the use of long orthotic devices which fixed the joints of his hips, knees and ankles in a way similar to that used to move an artificial limb prosthesis).

The neurorehabilitation methods developed by Vojta and Bobath are based on the double stimulation of the muscle sets, through the nervous reflex and muscle-muscle stimulus connected like the links in a chain. The efficient stimulus of the previously stimulated muscle fiber adds to the neurological impulse received by the re-innerved muscle fiber. This phenomenon enables the reconstitution, by retrograde stimulus, of the ability to move the whole body from the neck to the lower limbs in a manner resembling a child's neurodevelopment.

Patient 2 received neurorehabilitation similar to that Patient 1 received. However, the patients in such programs reach a plateau in their muscle development after about six months. Thus, the subsequent rehabilitation programs are designed to maintain the patient's achievements as well as to optimize use of the recovered functions.

It is evident that Patient 2 developed new motor and sensory function after the reinnervation of new muscle groups. The improvements in her condition are attributable to the combination of the autoimmune T-cell and stem cell therapy in combination with the neurorehabilitation program.

The results described in this Example 5 indicates that this combined immunotherapy scheme induces repair of the areas affected by CNS acute and chronic trauma with an approach which is minimally invasive and presents minimal or no adverse events.

### Example 6 - Treatment of ALS.

Previously, ALS patients have been treated with vaccination with T-cells reactive against motor neurons. Nine patients were treated. Seven of the nine patients responded to therapy and after nine years, two of the seven responders are still alive an neurologically stable.

In an attempt to improve these results, the stem cell therapy of the present invention was combined with this prior therapeutic method.

Four ALS patients were recruited. Autoimmune T-cells reactive against motor neurons were isolated using the protocol of Example 1. The population of autoimmune T-cells were divided. Half were frozen for later use and the other half was infused into the patient by I.V. drip.

After one month, MSCs were isolated from the patients using the protocol of Example 2 and co-cultured with the unfrozen autoimmune T-cells. Differentiation of the stem cells into motor neurons was assessed by assaying Nestin and Tubulin III immunostain. The remaining AMT were administered by I.V. Forty-eight hours later, the differentiated MSCs were administered to the patient by I.V. infusion.

Neurological evaluations were performed following ALS Functional Rating Scale-R. Hand force and respiratory function were also assessed. Disease progression was haled in three of the four patients. Two of the four patients showed improved gait, hand force and respiratory capacity. Adverse effects were minimal.

### Example 7 - General Methodology.

The following protocols were observed for all of the Examples described above.

### Preventative Monitoring of Patient Receiving Autoimmune T-cells

A program of preventive measures was established to monitor patients receiving autoimmune T-cells to limit adverse reactions. The program included the following measures. First, during the treatment the patients' general clinical conditions (temperature, blood pressure and urinary output) were monitored. Second, 500 mg ASA and 10 mg loratadine were administered to the patients in 500 cc saline solution in parallel to the cell suspension. Third, if the urinary output was reduced, the patients would receive 10 mg furosemide I.V. Fourth, if the patient's blood pressure dropped below 90 mm Hg, the patient would receive 500 to 1000 cc saline solution at free dripping. Fifth, if nausea or vomiting was observed, the patient would receive 10 mg metroclopramide I.V. This program was developed based on previous cell therapy experience with similar though different treatments. As long as the program was followed, no other adverse events were observed.

### Quality control and Quality Assurance of the Cell Process.

All methods and protocols involving cell manipulations were performed by highly trained personnel, according the AABB (American Association of Blood Banks) Standards for a clean room that follow the GTP (Good Tissue Practices) final rules of the FDA (37 C.F.R. Part 1271 and the cGMP)

Microbiological studies were conducted to detect contamination by bacteria, fungi or viruses on (i) the samples taken from the bone marrow cell suspension, (ii) the aphaeresis products, (iii) the selected cells and (iv) the final product of each culture. If any sample proved positive for contamination, the culture was discarded and the procedure was repeated.

Flow cytometry analyses were performed to corroborate the identity of the cell suspensions (i) at the beginning of each culture, (ii) at each critical point of the cellular process and (iii) upon production of the final product.

The identification of the cell suspension of the MSCs and differentiated stem cells was primarily based on the cytological appearance of the tissue culture by observation through the phase contrast inverted microscopy according to the following criteria: MSCs
- at least 90% of the cell observed in a 10x highly magnified field must have had homogeneous cytoplasm with at least 4 nucleoli per nucleus; and differentiated stem cells
- after 48 hours of co-incubation, 80% of the observed cells should have had homogeneous granular cytoplasm (polarized or not) and a single large nucleus with not more than 2 prominent nucleoli.

### Patient Safety Evaluations

The safety evaluations were performed in accordance with IND regulations 21 C.F.R. 312.32. Evaluations were performed (i) prior to the administration of the autoimmune T-cells, (ii) 24 hours after the administration of the autoimmune T-cells, (iii) on the day of the intra-arterial infusion of the CNS differentiated MSCs, and (iv) 24 and 96 hours after the intra-arterial infusion. Thus, both early and late reactions and avoid toxic complications related to the administration of the different therapeutic agents were monitored.

A complete clinical exam was performed at each evaluation consisting of the recording of the patient's weight, vital signs; as well as records of the patient's clinical, neurological, cardio respiratory, digestive, renal and endocrine activity.

Laboratory determinations, performed at these evaluations enabled us to study the functions of bone marrow, liver and kidneys as well as the most significant metabolic parameters, infection or systemic inflammatory activity, electrolytic balance and myocardial function through EKG. A total of one hundred and twenty seven items were assessed at each evaluation.

Adverse events were evaluated by the "Common terminology criteria for Adverse Events (AE), 2004" developed by NIH. The definitions are available on the web at ctep.cancer.gov in the folder reporting/ctc.html

Although the patients did not develop major adverse events, precautions were taken. If patient developed symptoms of acute hypersensitivity, anaphylaxis, or Systemic Inflammatory Response Syndrome (SIRS), they would have been treated with the appropriate supportive care, depending on the severity of the reaction, including admission into an Intensive Care Unit.

If any severe adverse events (SAE) had occured, the treating physician, in consultation with the IRB, would make any determinations as regards the continuation of therapy.

### ADDITIONAL REFERENCES

The following references are hereby incorporated by reference for all that they teach.
1. Moalem G, Leibowitz-Amit R, Yoles E, Mor F, Cohen IR, and Schwartz M. Autoimmune T cells protect neurons from secondary, degeneration after central nervous system axotomy. Nature Medicine. 1999; 5:49-55.
2. Yoles E, Hauben E, Palgio O, Agranov E, Gothilf A, Cohen A, Kuchroo V, Cohen IR, Weiner H and Schwartz M. Protective Autoimmunity is a Physiological Response to CNS Trauma. J Neurosci 2001; 21: 3740-8.
3. Kerschensteiner M, Gallmeier E, Behrens L, Leal VV, Misgeld T, Klinkert WE, Kolbeck R, Hoppe E, Oropeza-Wekerle RL, Bartke I, Stadelmann C, Lassmann H, Wekerle H, Hohlfeld R. Activated human T cells, B cells, and monocytes produce brain-derived neurotrophic factor in vitro and in inflammatory brain lesions: a neuroprotective role of inflammation? J Exp Med. 1999; 189:865-70
4. Leone AM, Rutella S, Bonanno G, Abbate A, Rebuzzi AG, Giovannini S, Lombardi M, Galiuto L, Liuzzo G, Andreotti F, Lanza GA, Contemi AM, Leone G, Crea F. Mobilization of bone marrow-derived stem cells after myocardial infarction and left ventricular function. Eur Heart J. 2005 Jun; 26 (12):1196-204.)
5. Moviglia G.A., Varela G., Gaeta C.A., Brizuela J.A., Bastos F., Saslavsky J. Autoreactive T Cells Induce In Vitro Bone Marrow Mesenchymal Stem Cells Transdifferentiation to Neural Stem Cells. Cytotherapy 2005 (sent to publication)
6. Moviglia GA, Iraola N, Varela G, Bertolotto R, Hirsch J, Varela O, Palleros CA, Lopez AM & Shuster GS. T Cell Vaccination (TCV) for Secondary Chronic Progressive Multiple Sclerosis (CPMS), Associated to Interferon (IFN) and Total Plasma Exchange (TPX) Pre-Treatment. Benzon Symposium No 49, Multiple Sclerosis: Genetics, Pathogenesis and Therapy. August 19-22, 2002, Copenhagen, Denmark.
7. US Department of Health and Human Services. FDA. CDER. CBER. ORA. Guidance for Industry Sterile Drug Products produced by Aseptic Processing. Current Good Manufacturer Practices. September 2004 cGMP.
8. Prockop DJ, Gregory CA and Spees JL. One Strategy for Cell and Gene Therapy: Harnessing the Power of Adult Stem Cells to Repair Tissues. Proc. Natl Acad. Sci. 2003; 100 (S1):11917-23.
9. Ehrhard PB, Erb P, Graumann U, Otten U. Expression of nerve growth factor and nerve growth factor receptor tyrosine kinase Trk in activated CD4-positive T-cell clones. Proc Natl Acad Sci USA. 1993; 90:10984-8.
10. Moalem G, Gdalyahu A, Shani Y, Otten U, Lazarovici P, Cohen I, Schwartz M. Production of neurotrophins by activated T cells: implications for neuroprotective autoimmunity. J. Autoimmun. 2000;15:331-45
11. Muhallab S, Lundberg C, Gielen AW, Lidman O, Svenningsson A, Piehl F, Olsson T. Differential expression of neurotrophic factors and inflammatory cytokines by myelin basic protein-specific and other recruited T cells infiltrating the central nervous system during experimental autoimmune encephalomyelitis. Scand J Immunol. 2002; 55:264-73.
12. Chu K, Kim M, Jeong SW, Kim SU, Yoon BW. Human neural stem cells can migrate, differentiate, and integrate after intravenous transplantation in adult rats with transient forebrain ischemia. Neurosci Lett. 2003 Jun 5; 343(2):129-33.13.
13. Kang K-S, Kim SW, Oh YH, Yu JW, Kim K-Y, Park HK, Song C-H, Han H. A 37-year-old spinal cord-injured female patient, transplanted of multipotent stem cells from human UC blood, with improved sensory perception and mobility, both functionally and morphologically: a case study. Cytotherapy Vol 7 N°4 September 2005 368 - 373
14. Satake K, Lou J, Lenke LG. Migration of Mesenchymal stem cells through cerebrospinal fluid into injures spinal cord tissue. Spine 2004; 29: 1971-9
15. Sipski M, Jackson AB, Gomez- Marin O et al. Effects of gender on neurogenic and functional recovery alter spinal cord injury. Arch Phys Med Rehabil 2004; 85: 1826-36.
16. Myckatyn TM, Mackinnon SE, Mc Donald JW. Stem cell transplantation and other novel techniques for promoting recovery from spinal cord injury. Transplant Immunol2004; 12: 343-58.
17. Ankeny DP, Mc Tigue DM, Jakeman LB. Bone marrow transplants provide tissue protection and directional guidance for axon after contusive spinal cord injury in rats. Exp Neurol 2004; 190: 17-31.
18. Gluckman E, Rocha V, Boyer-Chammard A et al. Outcome of cord blood transplantation from related and unrelated donors. N Engl J Med 1997; 337: 373-81.
19. Tondreau T, Meuleman N, Delforge A, Dejeneffe M, Leroy R, Massy M, Mortier C, Bron D, Lagneaux L. Mesenchymal stem cells derived from CD133-positive cells in mobilized peripheral blood and cord blood: proliferation,Oct4 expression, and plasticity. Stem Cells. 2005 Sep;23 (8): 1105-12.
20. Gallacher L, Murdoch B, Wu DM, Karanu F, Keeney M, Bathia M. Isolation and characterization of human CD34- Lin - CD34 + Lin - hematopoietic stem cells using cell surface markers AC133 and CD7. Blood, 2000 May; 95 (9): 2813-20.
21. Jeme,NK. Towards a network theory of the immune system. Ann. Immunol. (Inst. Pasteur) 1974; 125C: 373-389,
22. Moalem G, Leibowitz-Amit R, Yoles E, Mor F, Cohen IR and Schwartz M Autoimmune T cells protect neurons from secondary degeneration after central nervous system axotomy. Nat Med 1999 5:49-55.

## Claims

1. A method of inducing a population of stem cell to differentiate into a target cell type comprising:
a) providing a population of stem cells capable of differentiating into the target cell type;
b) contacting the population of stem cell with a population of autoimmune T-cells reactive against the target cell type for a time period sufficient to induce differentiation of the population of stem cells into the target cell type.

2. The method of claim 1 wherein the target cell type is selected from the group comprising: central nervous system cells, pancreatic cells, and heart muscle cells.

3. The method of claim 1 wherein the population of stem cells comprise mesenchymal stem cells, adipose-derived stem cells or hematopoietic stem cells.

4. The method of claim 1 wherein the population of stem cells comprise mesenchymal stem cells.

5. A method oftreating a subject with organ, tissue or cell damage comprising:
a) providing a population of stem cells capable of differentiating into the organ cells, tissue cells or cells;
b) contacting the population of stem cell with a population of autoimmune T-cells reactive against the organ cells, tissue cells or cells for a time period sufficient to induce differentiation of the population of stem cell into a differentiated population of the organ cells, tissue cells or cells.
c) administering a therapeutically effective amount of the differentiated population of the organ cells, tissue cells or cells to the subject.

6. The method of claim 5 further comprising administering a therapeutically effective amount of the population of autoimmune T-cells to the subject prior to step (c).

7. The method of claim 6 wherein the organ, tissue or cell damage is neural damage.

8. The method of claim 7 wherein the neural damage is spinal cord damage, amyotrophic lateral sclerosis, Parkinson's disease, or Alzheimer's disease.

9. The method of claim 6 wherein the population of stem cells comprise mesenchymal stem cells, adipose-derived stem cells or hematopoietic stem cells.

10. A method oftreating a subject with organ, tissue or cell damage comprising:
a) administering to the subject a therapeutically effective amount of a population of autoimmune T-cells reactive to the organ cells, tissue cells or cells; and
b) administering to the subject a therapeutically effective amount of a population of stem cells.

11. The method of claim 10 wherein the population of stem cells is contacted with a population of autoimmune T-cells reactive against the organ cells, tissue cells or cells for a time period sufficient to induce differentiation of the population of stem cell into a population of the organ cells, tissue cells or cells before administering to the subject in step (b).

12. The method of claim 11 wherein the organ, tissue or cell damage is neural damage.

13. The method of claim 12 wherein the neural damage is spinal cord damage, amyotrophic lateral sclerosis, Parkinson's disease, or Alzheimer's disease.

14. The method of claim 11 wherein the population of stem cells comprise mesenchymal stem cells, adipose-derived stem cells or hematopoietic stem cells.

15. A composition comprising a population of stem cells capable of differentiating into the target cell type in contact with a population of autoimmune T-cells reactive to at least one protein expressed by the target cell type.

16. The composition of claim 15 wherein the population of autoimmune T-cells is reactive to central nervous system cells, pancreatic cells, or heart muscle cells

17. The composition of claim 15 wherein the population of stem cells comprises mesenchymal stem cells, adipose-derived stem cells or hematopoietic stem cells.

18. The composition of claim 15 wherein the population of stem cells comprises mesenchymal stem cells.
